# EUROPEAN PATENT APPLICATION

(11) **EP 4 325 197 A1**
(43) Date of publication of application: **21.02.2024**
(21) Application number: 22191181.1
(22) Date of filing: 19.08.2022
(51) Int. Cl.: G01N 1/10, G01N 1/16, C12Q 1/04, C12Q 1/24, B01L 3/00, G01N 33/50, G01N 33/18, G01N 1/14

(54) **A SAMPLING DEVICE AND A METHOD FOR COLLECTING MICROORGANISMS FROM THE ENVIRONMENT BY CHEMOTAXIS**

(71) Applicant: ETH Zurich, 8092 Zurich (CH); University of Technology Sydney, Ultimo, New South Wales 2007 (AU)
(72) Inventor: STOCKER, Roman, 8006 Zürich (CH); CLERC, Estelle, 8006 Zürich (CH); SEYMOUR, Justin, Hornsby, 2077 (AU); RAINA, Jean-Baptiste, Glebe, 2037 (AU); ARNOLD, Michael, 8006 Zürich (CH); ENGLER, Christian, 28215 Bremen (DE)
(74) Representative: Maikowski & Ninnemann Patentanwälte Partnerschaft mbB

(57) **Abstract**

The present invention relates to a sampling device for collecting microorganisms from aquatic environments by chemotaxis comprising: a housing, at least one electronic control unit interacting with the housing / connected to the housing; and at least one rectangular cast that can be arranged within the housing wherein the cast is designed to receive at least one assay plate, preferably multiple assay plates (8 or more); wherein the at least one assay plate comprises at least one well, preferably up to 20 or more wells, filled with at least one chemoattractant compound, and wherein each well is connected to the outside by at least a port; wherein the cast is further designed to receive at least one sealing plate at least partially covering the at least one assay plate, wherein the sealing plate is designed to be moveable along the at least one assay plate between a first position, wherein the at least one sealing plate seals the port of the at least one well of the assay plate (sealing position / closed position), and a second position, wherein the at least one sealing plate opens the port of the at least one well of the assay plate (open position), and wherein the cast is connected to the at least one electronic control unit. The present invention relates also to a method for sampling microorganisms using said sampling device in any type of environment containing a liquid phase.

## Description

The present invention relates to a sampling device for collecting microorganisms from differen environments, in particular aqueous environments, by chemotaxis and to a method using such sampling device.

### Description

More than two million tons of sewage, industrial and agricultural waste is released every day into water bodies around the world (UN WWAP, 2003). This pollution is plaguing our environment as well as affecting animal and human health. The pollutants released in the environment are not only produced in high amounts in the industrial sector but are also directly derived from human waste. Their efficient treatment is problematic as most state-of-the-art disposal methods (incineration, landfill disposal, filtration and adsorption, chemical degradation) are either not sufficiently performant in capturing all pollutants from production sites or preventing them to enter the natural environment, or they are also often highly harmful to the environment by increasing atmospheric CO₂ concentrations, producing toxic by-products or directly affecting human and animal health.

The field of novel bacteria and enzymes discovery has been aiming at providing sustainable bio-based solutions to respond to this problem. However, biodiscovery encounters many fundamental biological and technical issues that hinders the success rate and application potential of novel findings, currently leaving biological interventions in pollution management as a promising yet unreliable solution.

The most traditional bacterial discovery methods in the environment usually consist in the collection of a large amount of material (e.g. water, soil) in an untargeted manner, bringing back the samples to the laboratory and treating them by multiple series of highly time-taking selective culturing steps and activity-based assays which often is biased for the growth of a limited number of microbial species (Eilers et al. Culturability and in situ abundance of pelagic bacteria from the North Sea. Applied and environmental microbiology, 66(7), 3044-3051; 2000).().

More recent developments of advanced growth devices such as a micro-Petri dish (Ingham et al., The micro-Petri dish, a million-well growth chip for the culture and high-throughput screening of microorganisms. Proc. Natl. Acad. Sei. 104, 18217-18222, 2007), a boxed glass culture dish (Yunnan Inst. Tobacco Agri. Sci., CN112375687A), or the GALT Prospector^{™} system have nevertheless enabled isolation of novel microbes in recent years, yet remain laboratory-based techniques. The IChip (NovoBiotic Pharmaceuticals; Berdy et al., In situ cultivation of previously uncultivable microorganisms using the IChip. Nat. Protoc. 12, 2232-2242; 2017) is an in situ culturing technology that has been able to generate a large bacteria collection and discover novel antibiotics (WO2014089053), but the starting bacterial inoculum remains unselective. Conversely, another screening device has been developed for in situ soil isolation but is restricted to oil-degrading strains (CN107118966A).

Advances in genomics and bioinformatics have also allowed computational targeted techniques such as genome mining to discover new enzymes in pooled or individual bacterial genomes (Ferrer et al., Mining enzymes from extreme environments. Curr. Opin. Microbiol. 10, 207-214, 2007; Ferrer et a/., Metagenomices for mining new genetic resources of microbial communities. Microb. Physiol. 16, 109-123, 2009).

However, these technical and computational approaches are not only very time-taking or expensive (Popovic et al., Metagenomics as a tool for enzyme discovery: Hydrolytic enzymes from marine-related metagenomes. Advances in experimental medicine and biology, 883, 1-20. 2015), but are also oblivious of the natural bacterial dynamics happening in situ at the microscale (Stocker, Microbes see a sea of gradients. Science 338, 628-633, 2012) limiting the rate and outcome of novel biodiscovery as well as the probability of finding highly metabolically performant bacteria.

Individual microbial behaviors happening at the size of a single microbe, the microscale, control the biogeochemistry and productivity of natural ecosystems, and the rate at which each single microbes access substrates they need from their surroundings, depends upon the spatial distribution of substrates and the capacity of cells to exploit ephemeral nutrient hotspots (Azam, F. Microbial control of oceanic carbon flux: The plot thickens. Science 280, 694-696 (1998); Stocker, R., Marine Microbes See a Sea of Gradients. Science 338, 628-633 (2012); Clerc, et al., Survival in a sea of gradients: Bacterial and archaeal foraging in a heterogeneous ocean. in The Marine Microbiome (eds. Stal, L. J. & Cretoiu, M. S.) vol. 3, 47-102 (Springer International Publishing, 2022). These microscale behaviors and heterogeneity are tremendously important as they do eventually impact large-scale geo-chemical processes, such as the carbon cycle. However, the immense resources and knowledge that microscale behaviors provide remain largely inaccessible by traditional bulk sampling. Our method overcomes this major challenge by accelerating biodiscovery through an unprecedented in situ screening technique for bacteria and enzymes discovery by leveraging the microscale bacterial behavior called chemotaxis, the ability of heterotrophic bacteria and archaea to move up or down chemical gradients and exploit transient nutrient patches in a heterogeneous environment.

The closest finding is an in situ device for microorganisms detection called Fungialter LTD (WO2017207756 A1 and WO2019154997 A1), acting as a pests control system in agriculture and horticulture. The device delivers an attractant into a growth substrate or water to direct responding microorganisms (i.e. fungi) to a detector for signalization of their presence. Fungialert aims to signal the presence of pathogens and can only target one compound at a time and is thus not suited for a high-throughput capture of microorganisms.

Conversely, chemotaxis appears to be widely used in microfluidic devices to investigate target species, moieties or compounds yet are limited to laboratory settings, such as in "Microfabricated Cellular Traps Based On Three-Dimensional Micro-Scale Geometries" (WO2006025858 A2).

The object of the present invention was therefore to overcome the existing hurdles and problems and to provide an approach to use chemotaxis for high-throughput bacterial capture, isolation and their further use in biodegradation and industrial processes.

This object has been solved by providing a sampling device with the features of claim 1 and a method with the features of claim 11.

Accordingly, a sampling device for collecting microorganism from a liquid, in particular an aqueous environment by chemotaxis is provided, wherein the sampling device comprises:
- a housing,
- at least one electronic control unit interacting with the housing, in particular connected to the housing; and
- at least one cast, in particular a rectangular cast, that can be arranged within the housing
   wherein the cast is designed to receive at least one assay plate, preferably multiple assay plates;
   wherein the at least one assay plate comprises at least one well, preferably up to 20 or more wells, filled with at least one chemoattractant compound, and preferably at least one water negative control, and
   wherein each well is connected to the outside by at least a port;
   wherein the cast is further designed to receive at least one sealing plate at least partially covering the at least one assay plate,
   wherein the sealing plate is designed to be moveable, in particular movable along the at least one assay plate, between
      a first position, wherein the at least one sealing plate seals the port of the at least one well of the assay plate (sealing position / closed position), and
      a second position, wherein the at least one sealing plate opens the port of the at least one well of the assay plate (open position),
   wherein the cast further contains at least one motor, in particular a pressure compensated motor, for moving the at least one sealing plate along the at least one assay plate between the first position and the second position, and
   wherein the cast is connected to the at least one electronic control unit.

Thus, a sampling device is provided that allows screening for bacteria of interest based on their chemotactic preference to a substance in a wide range of environments through the use of specifically designed assay plates in combination with deployment enclosure.

The sealing plate prevents unwanted contamination during the traveling time through the water column by sealing the ports of the assay plate. At the target depth, the sealing plate opens for a predetermined amount of time and allows for the chemicals to diffuse and chemotaxis to occur, capturing responding bacteria inside the well of the assay plate. This is achieved by allowing the sealing plate to be moved between a first (closed) position when moving the sampling device through the aquatic environment and a second (open) position once the targeted position is reached. Once the sampling is completed, the sealing plate moves back from the second (open) position back to the first (closed) position for capturing the responsive microorganisms and moving the sampling device back to the starting point (i.e. forward-backward-movement).

The electronics, secured on the side of the housing, allow for a fully stand-alone deployment.. It possesses its own drop weight and needs to be actuated manually. However, other constructions may also be used. For example, a suitable construction could be based on a modified sampler or any other type of deployment mechanism. The present sampling device enables the implementation of a new biodiscovery method using chemotaxis that bacteria use to direct themselves in a targeted manner to nutrient sources of preference. This allows for screening for the bacterial activity of interest and therefore accelerates the rate of biodiscovery, what traditional bulk sampling methods, advanced bacterial growth technologies and genome-based targeted enzyme discovery cannot do. Due to the optimized sampling device it is now possible to sample environments that are usually difficult to access, opening new avenues for novel biodiscovery. Additionally, both of the applied microtechnology and sampling device are easily scalable and can reach a high-throughput efficacy therefore reducing the costs of the procedures and biodiscovery.

The present sampling device and method provide an approach for discovering new metabolically-potent bacteria in the environment and exploit them to degrade industrial and environmental pollutants, but not only. Other applications may include the discovery of bacteria and their enzymes that degrade, modify/transform or accumulate any type of chemicals both in industrial settings, or environments, the discovery of bacteria producing active substances of industrial or pharmaceutical uses, and/or the discovery of any other types of swimming microorganisms (phyto and zooplankton) with the above-mentioned abilities.

The present device and method enable a targeted search to a wide range of environments, including extreme ones, allowing to go beyond the current limit of bioprospecting and enabling the capture of bacteria and their enzymes with novel or enhanced metabolic activities.

Indeed, bacteria detected by applying the present device and method may be suitable to break down or transform any complex substances, which are often a characteristic of recalcitrant pollutants but not limited to oil, polychlorinated biphenyls (PCBs), polyfluoroalkyl substances (PFAS) or microplastics. Thus, bacteria and/or cocktails of their enzymes can be provided for targeted, cost-efficient and eco-friendly degradation of specific pollutants and other applications as mentioned above. This approach can efficiently address the degradation of harmful chemicals in industrial sources, increases performance of pollutants removal in wastewater treatment plants, preventsi additional pollution to be leaked in the environment, and may allow to intervene directly in the environment in cases of emergency (i.e. oil or chemical spill).

The sampling device and sampling method have a broad range of applications in multiple industries, including pharmaceuticals, food, agriculture, chemicals, biotechnology, governmental, and more. By targeting a specific compound of mixture of compounds, it is possible to deliver highly performant single bacteria and/or enzyme or bacteria and/or enzymes cocktails derived from the specific bacterial consortium. It allows for bacteria and enzymes discovery on demand of customers and their specific needs, while providing subsequently custom single bacteria and/or enzyme or bacteria and/or enzymes cocktails.

The different features of the sampling device are now described in more detail.

### Cast

As described, the cast holds the assay plates and contains a pressure compensated motor allowing for autonomous activation at depth (based on a sensor) and a connection to the electronic enclosure. The cast also is designed to hold or keep the at least one assay plate, preferably more than one assay plate, most preferably more than 4, such as 8 assay plates or more in its position.

In an embodiment, the at least one cast is made of pressure and corrosion resistant material, in particular is made of polyoxymethylene (POM). Besides being a pressure and corrosion resistant material, the selected material does not release chemicals repulsing bacteria. However, any other inert material, with the required properties may be suitable.

As mentioned, the cast has a rectangular, elongated shape. Other shapes are also applicable, such as square, diamond, cross. The size of the cast is chosen in a manner to deploy an optimal number of assay plates and avoid at the same time the system being too heavy.

The cast is designed in form of a frame with two opposite long edge sides and two opposite short edge sides and two opposite surfaces. The frame edge sides are designed to create a (rectangular) space with two (rectangular, planar) opposite openings on both opposite frame surfaces.

At least one surface side of the cast frame is designed to receive at least one sealing plate (e.g. can be covered by at least one sealing plate). In this case, the other surface side of the cast frame may be covered by any conventional means. In another embodiment, both surface sides of the cast frame can be designed to receive each one at least one sealing plates.

The frame space or frame content of the cast frame is designed to receive and hold the assay plates. In one embodiment the cast frame may comprise a type of tracks to and any other suitable means to hold the assay plates.

The frame cast may be designed to receive assay plates through one or both opposite openings of the cast frame.

The speed of the motor should be chosen such not to create turbulence upon motion.

### Assay plates

The assay plates have been described previously (Lambert., et al. A microfluidics-based in situ chemotaxis assay to study the behaviour of aquatic microbial communities. Nat. Microbiol. 2, 1344-1349 (2017) and are called In Situ Chemotaxis Assay (ISCA). By using this approach bacteria are attracted in situ based on their chemical preferences and captured directly in their natural environment for further laboratory culturing and characterization. Chemotactic responses can be quantified by counting bacteria present in the wells using flow cytometry, and the sampled bacteria can be further characterized by extracting their DNA, sequencing them and directly isolating them.

As mentioned, the at least one cast contains one or multiple assay plates. Each assay plate comprises at least one well, preferably up to 20 wells, filled with at least one chemoattractant compound and at least one negative control, such as ultrafiltered seawater. The number of wells of each assays plate can vary and is not limited to 20 wells. Thus, also the numbers of wells per row can vary and is not limited to 5. The number of wells solely depends on the overall size of the assay plate and sampling device.

Each well of the assay plate is connected to the outside environment by at least a port. In a preferred embodiment, the port of each well of the at least one assay plate is offset from the center of the well.

The port allows the chemoattractant to diffuse out of the well into the environment, preferably an environment with an aqueous phase, such:
- Marine ecosystems: including any depth of seas and oceans water column, mudflats, seagrass meadows, mangroves, intertidal zones, salts marshes, coral reefs, lagoons, estuaries hydrothermal vents, backwaters;
- Freshwater ecosystems: including lakes, rivers, ponds, streams, springs, bogs, wetlands, marshes, snow and ice, glaciers and ice caps;
- Soil and groundwater;
- Industrial and man-made environments: including wastewater treatment plants, freshwater reserves, rainwater and stormwater retention tanks, bioreactors and chemical tanks, aquaria, swimming pools.

Thus, the sampling device may be deployed in any type of environment containing at least an aqueous phase

This results in a chemical microplume extending for example 1-2 mm above each well within a preferable deployment time of, but not limited to, 1 to 4h.

In turn, the microorganisms attracted by the selected chemoattractant(s) can enter the well through the port. The chemotactic responses is subsequently quantified by counting bacteria present in the wells using flow cytometry, and further characterized by extracting and sequencing their DNA, and directly isolating them. This will be described in more detail further below.

The assay plate device is made of inert materials, is single-use and is fabricated using a standard soft lithography workflow, based on a mould created using three-dimensional printing. The material used for the assay plate(s) may be glass, polydimethylsiloxane (PDMS) or polycarbonate. However, any other inert material, in particular inert plastic material with the required properties may be suitable.

### Sealing Plate

As described previously, at least one sealing plate is provided for at least partially covering the at least one assay plate. The at least one sealing plate is placed and mounted in a movable fashion on or to the cast, in particular cast frame. The at least one sealing plate is preferably mounted movably on one side of the cast, in particular on one surface side of the cast frame as previously described.

In one embodiment, at least two sealing plates are provided on the opposite sides of the cast, in particular opposite surface sides of the cast frame, as previously described.

Said sealing plate is designed to move along or over the surface of the at least one assay plate between a first position, wherein the at least one sealing plate seals the port of the at least one well of the assay plate (sealing position / closed position), and a second position, wherein the at least one sealing plate opens the port of the at least one well of the assay plate (open position). The sealing plate is freely movable between both positions for allowing closed and open position of the assay plate whenever required.

The sealing plate seals the ports of each well of the assay plate with high precision and slides autonomously at the predetermined sampling depth to release the chemical plume. Thus, the sealing plate prevents unwanted contamination of the wells of the assay plate during the traveling time through the water column by sealing said wells of the assay plate.

The overall design of the sealing plate including thickness and angles to avoid turbulences. The sealing plate may be designed in a grid-like manner with alternating slit-forming gaps for open position and bridges for sealed position. The width of the slits or gaps is chosen to enable optimal chemical diffusion and chemotaxis.

The bridges are provided with evenly spaced elements for sealing the port of the well. The sealing elements are arranged in a line on said bridges, which is parallel to each row of wells of the assay plate, when the sealing plate covers the assay plate.

The number of said sealing elements on each bridge is identical to the number of wells in each row of the assay plate. For example, if an assay plate has 5 wells per row then 5 sealing elements are provided. This ensures that each port of the wells of one row in the assay plate can efficiently be sealed.

The sealing elements may have a spheric shape and are embedded in holes within the sealing plate. Size and shape of the holes correspond to size and shape of the embedded sealing means. In a preferred embodiment the sealing means are provided in form of a rubber sphere.

The rubber spheres are embedded in the plate and provide an efficient seal while in the closed position.

The at least one sealing plate may be made of the same material as the cast, i.e. a pressure and corrosion resistant material, in particular is made oftitanium. Other types of material with the same required properties may be used.

### Housing

The housing is a modified Niskin bottle for hosting the sealing plate and cast and for maintaining a low-flow environment during the deployment. The bottle is also made to be connected to the electronics control unit, preferentially screwed to a frame attached to its side

A Niskin bottle is a device for obtaining samples of water at a specific depth. The 'bottle' is a tube, usually plastic to minimize contamination of the sample, and open to the water at both ends. Each end is equipped with a cap which is either spring-loaded or tensioned by an elastic rope. The bottle is closed by Tthe action of a messenger weight that trips both caps shut and seal the tube. A reversing thermometer may also be carried on a frame fixed to the Niskin bottle.

An embodiment of the Niskin bottle uses actuated valves that may be either preset to trip at a specific depth detected by a pressure switch, or remotely controlled to do so via an electrical signal sent from the surface. This arrangement conveniently allows for a large number of Niskin bottles to be mounted together in a circular frame termed a rosette.

The Niskin bottle used in the present case was modified to be able to host the cast containing the assay plates and the sliding plates, and to favor its attachment to the electronic control unit on its side.

In a first modification, the clamping mechanism of the bottle was modified to be able to keep the lids open while mounting the cast inside the housing. For this purpose, a rubber-based rope is inserted through a hole situated in the middle of the top lid, navigated through the bottle and taken out through the hole made in the bottom lid of the bottle. The rope is then pulled and kept under tension by fixing it to the bottom lid.

In a second modification, a hole was drilled in the side of the bottle to host a cable gland, allowing for the connection of the cast to the external electronic control unit on the side of the housing.

### Electronic control unit

In an embodiment, the at least one electronic control unit is secured on the side of the housing in a titanium enclosure allowing for a fully stand-alone deployment. However, the electronic unit may also be placed at any other suitable location.

The electronic control unit contains the electronics controller and the batteries. The controller is used to action the motor moving the sliding plate (sealing plate). The controller retrieves deployment parameters from an SD card and starts the deployment sequence when the preset depth is reached. While the device is under water, the controller logs sensor data to a csv file on the SD card, continaing the following parameters: date, time, depth, slider state, voltage, current, gyro X, gyro Y, gyroZ, temperature.

The controller is placed inside the watertight titanium enclosure and switched on by a watertight plug switch. The electronic control unit is connect to the motor contained in the cast within the modified Niskin bottle by a watertight connector, that goes through a cable gland in the side of the modified Niskin bottle.

The electronic control unit contains a battery pack (12V to 24V).

The sampling device as described may be employed in a method for collecting bacteria from liquid environments by chemotaxis, wherein the method comprises the following steps:
- Providing at least one assay plate with at least one well filled with at least one chemoattractant compound;
- Placing the at least one assay plate in the at least one cast, wherein the at least one assay plate is at least partially covered by the at least one sealing plate such that the ports of the wells of the assay plate are sealed / closed;
- Placing and securing the at least one cast with the at least one sealed assay plate in at least one housing, in particular a modified Niskin bottle;
- Descending the at least one housing with the at least one cast with the at least one sealed assay plate to a predetermined location, in particular predetermined depth in a liquid, in particular aqueous environment;
- Closing the housing once the predetermined location, in particular predetermined depth is reached, preferably by sending a heavy metal messenger to close the lids of the bottle;
- Moving the at least one sealing plate automatically from the closed position to the open position to open the ports of the wells of the assay plate;
- Keeping the ports of the wells open for a predetermined time for allowing bacteria to enter the wells of the assay plate through the ports (e.g. 1h to 4h or more);
- Moving the at least one sealing plate automatically from the open position to the closed position to close the ports of the wells of the assay plate after the predetermined sampling time; and
- Ascending the sampling device, in particular retrieving the sampling device to the surface and the samples are retrieved from the assay plates

It is to be understood that during descent the at least one housing (i.e. Niskin bottle) is open, while the ports of the wells of the assay plates are closed by the sealing plate. Once the predetermined depth is reached the at least one housing (i.e. Niskin bottle) is closed. This is done for example by sending down a weight along the wire to which the Niskin bottle is attached.

The electronics controller of the electronic control unit is triggered automatically when reaching the set depth or desired location and counts down a predetermined amount of buffer time to let turbulence settle in the housing (e.g. 15 min);

The sampling time, i.e. the time at which the ports of the wells are kept open for bacteria to enter the wells of the assay plate through the ports, is at least 1 hour, preferably at least 2 hours or more.

The chemoattractant compounds that may be employed in the present sampling method can be either single characterized compounds or uncharacterized chemical mixtures, either naturally occurring or man-made. Non exhaustively for example:
- Pharmaceuticals (e.g. antibiotics, hormones, analgesics, anti-depressants, antiinflammatories, anti-parasitics...)
- Food-derived substances or compounds used in food processing (e.g. additives, stabilizers, preservatives, ethanol, acrylamides...)
- Chemicals from chemical industry (e.g. dyes, paints, chlorinated solvents, detergents...),
- Agriculture-derived chemicals (e.g. pesticides, herbicides, insecticides, fungicides, fertilizers...)
- Wastewater and environmental water sampled in polluted location
- Packaging materials (e.g. plastics, petroleum compounds...)
- Heavy metals ( e.g. lead, cadmium, arsenic, mercury...)
- Unhealthful molecules and agents (toxins, pathogenic bacterial extracts...)

The present sampling method can be divided in three phases: phase A) descent of sampling device; phase B) sampling; phase C) ascent of sampling device.

### Phase A) Descent of sampling system:

The assay plates are placed in the cast in closed position to avoid any bacterial contamination during descent. The system is secured in the Niskin bottle, whose lids are in open position during the descent to the desired depth. The bottle is attached to a wire.

### Phase B) Sampling:

Once the target depth or desired location is reached, the Niskin bottle is first closed by sending down a weight along the wire. Some time (e.g. 15 min) is given for the remaining flow in the bottle to settle down and avoid turbulence-induced contamination. The sealing plate automatically moves and exposes the ISCA wells to the surrounding seawater, letting the chemicals diffuse from the wells. The wells are kept open for a predetermined duration to allow the bacteria to enter the wells (usually 1-2h or more).

### Phase C) Ascent of the sampling system:

After the predetermined exposure time is ended, the plate slides automatically and seals back the wells, capturing the chemotactic bacteria and making any bacterial contamination during the ascent impossible. The entire system is returned to the surface for sample collection, with the lids of the bottle closed to enable sampling of the depth or location-specific water.

The invention is explained in more detail by means of examples with reference to the figures. It shows:
- Figure 1 A: an assay plate for selectively capturing bacteria of interest;
- Figure 1 B, C: a modified Niskin bottle for housing the cast with assay plates;
- Figure 2 A, B: a sealing plate for sealing the wells of the assay plate
- Figure 2C: a cast for holding the assay plate and sealing plate
- Figure 3: sequence for deploying the sampling device,
- Figure 4: a diagram showing results of an in situ chemotactic response in a lake at 100 m deep.
- Figure 5: a diagram showing results of an in situ chemotactic response in the sea at 600 m deep.

### Example 1: assay plate

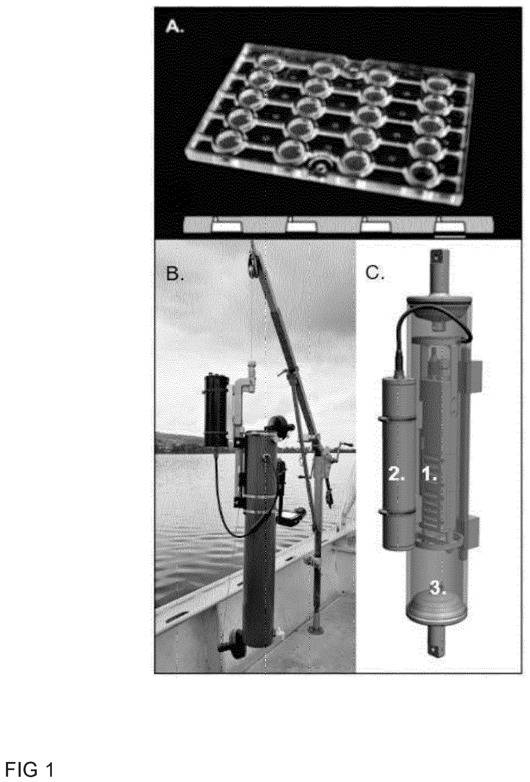

Fig 1A shows an assay plate suitable for the sampling device and sampling method. The assay plate is a robust and rapidly producible device that bridges the gap between laboratory-based microfluidics and traditional oceanographic methods, by providing an in situ system to interrogate microbial chemotactic behaviors.

The assay plate has the size of a credit card (75 mm × 47 mm), containing 20 wells that can be individually filled with putative chemoattractants (typically, 5 wells per compound, to ensure enough material for analyses). The device is made of inert material, is single-use and is fabricated using a standard soft lithography workflow, based on a mould created using three-dimensional printing.

The assay plate consists of a scalable array of 5 × 5 wells embedded in a polycarbonate slab, with each well connected to the outside environment, such as seawater or any other environment containing a liquid phase, by a port (0.8 mm diameter, 1.6 mm depth). Most of the wells are filled with chemoattractants, which diffuse out of the ports and into the surrounding seawater during deployment, resulting in chemical microplumes extending 1-2 mm above each well within a typical 1 to 2h of deployment. The chemical microplumes mimic transient nutrient patches. Chemotactic bacteria respond by swimming into the wells of the device. A row of 5 wells are always filled with ultrafiltered water originating from the same environment acting as negative control, that accounts for random microbial motility in the plate.

Chemotactic responses can be quantified by counting bacteria present in the wells using flow cytometry, and the sampled bacteria can be further characterized by extracting their DNA, sequencing them and directly isolating them.

While the original version of the device was made of glass and polydimethylsiloxane (PDMS), the device may also be made of polycarbonate. Polydimethylsiloxane (PDMS) is cast onto a 3D printed mould and cured overnight. The solid PDMS, containing multiple wells, is then excised and plasma-bonded onto a glass slide (100 mm × 76 mm × 10 mm).

### Example 2: sampling device

One embodiment of the present sampling device is shown in Figures 1 B, C.

A modified Niskin bottle (Fig. 1 C.3) (10L, 1150 mm x140 mm) hosts a cast (Fig. 1 C.1) (with up to 8 assay plates and 2 sealing plates) and maintains a low-flow environment during the deployment.

The sampling device (Fig. 1. B and C), holds a cast (608 mm × 56 mm × 48 mm) (Fig. 1 C.1) containing several assay plates copies, and enables to access remote environments. The system was tested in a pressure chamber that guarantees a successful functioning down to 4000 m depth.

The cast (Fig. 1 C.1) containing the assay plates and the sealing plate allows deployments at target depths. The sealing plate prevents unwanted contamination during the traveling time through the water column by sealing the ports of the assay plate. At the target depth, the sealing plate opens for a predetermined amount of time and allows for the chemicals to diffuse and chemotaxis to occur, capturing responding bacteria inside the wells of the assay plate.

The electronics housing (outside dimension: 110 mm × 322 mm) (Fig. 1 C.2) secured on the side of the bottle allows for a fully stand-alone deployment. The system possesses its own drop weight and needs to be actuated manually.

### Example 3: cast with sealing plate and assay plate

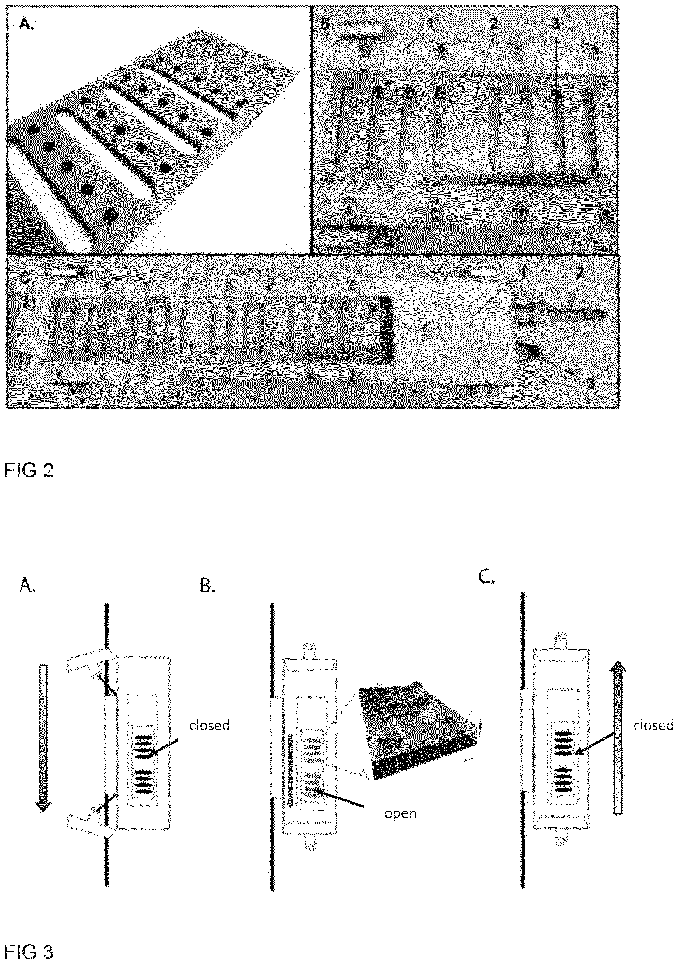

The cast (Fig. 2C) holds the assay plates and contains a pressure compensated motor allowing for autonomous activation at depth (based on a sensor) and a connection to the electronic enclosure (Fig. 2C.3). The cast (608mm × 56mm × 48mm) also maintains the two sealing plates (500mm × 50mm), situated on each side of the cast, in place.

The rectangular sealing plate (Fig. 2 A and 2B.2) seals the ports of the wells of the assay plates with high precision and slides autonomously at the predetermined sampling depth to release the chemical plume.

The sealing plate has the shape of a grid with alternating slits-forming gaps for open position (Fig. 2 B.3) and bridges for sealed position. The bridges are provided with evenly spaced rubber spheres as means for sealing the port of the well. The rubber spheres are embedded in the plate and provide an efficient seal while in the closed position.

The number of rubber spheres on each bridge is identical to the number of ports in each row of the assay plate. Fig. 1A shows a sealing plate with 5 rubber spheres for sealing 5 corresponding ports per row of wells of an assay plate.

### Example 4: Sampling method

Fig. 3 illustrates the sequence of deployment of the sampling device. The deployment can be divided in three phases: phase A) descent of sampling device; phase B) sampling; phase C) ascent of sampling device.

Descent of the sampling system to predetermined depth (Fig. 3A.):
1. The ISCAs are placed in the cast in closed position to avoid any bacterial contamination during descent.
2. The system is secured in the Niskin bottle, whose lids are in open position during the descent to the desired depth.
3. The bottle is attached to a wire.

### Sampling at predetermined depth: (Fig. 3B)

1. Once the target depth is reached, the Niskin bottle is first closed by sending down a weight along the wire.
2. Some time (e.g. 15 min) is given for the remaining flow in the bottle to settle down and avoid turbulence-induced contamination.
3. The sealing plate automatically moves and exposes the ISCA wells to the surrounding seawater, letting the chemicals diffuse from the wells.
4. The wells are kept open for a predetermined duration to allow the bacteria to enter the wells (usually 1-2h, or more).

### Ascent of the sampling system (Fig. 3C)

1. After the predetermined exposure time is ended, the plate slides automatically and seals back the wells, capturing the chemotactic bacteria and making any bacterial contamination during the ascent impossible.
2. The entire system is returned to the surface for sample collection, with the lids of the bottle closed to enable sampling of the depth or location-specific seawater.

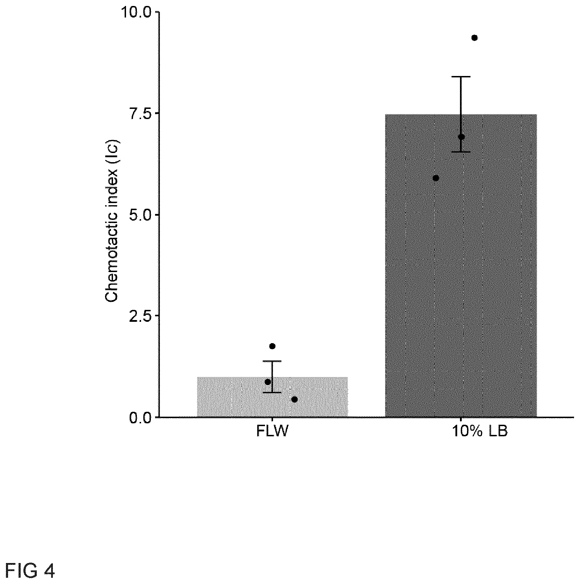

Figure 4 shows a diagram illustrating the in situ chemotactic response of a natural lake microbial population at depth (100 m) using the sampling device toward a rich medium (10% Luria-Bertani broth) after 60 min of deployment. The chemotaxis index /c represents the strength of attraction of the bacterial community towards a compound compared to a negative control of ultrafiltered lakewater. It is calculated by normalizing the cell counts obtained in the treatment wells by the mean cell count within the wells containing the filtered lakewater negative controls (FLW). Each treatment and control were tested in three ISCA replicates contained in the deployment system. Bacterial cells were quantified by flow cytometry (FLW = 5.73 ± 3.79 × 10³; 10% LB = 4.28 ± 0.92 × 10⁴ cells/mL). The concentration of LB medium tested at lake depth induced a chemotactic response significantly higher than the filtered seawater (FLW) controls. In all pairwise comparisons: t-test, p < 0.005; Error bars represent SEM

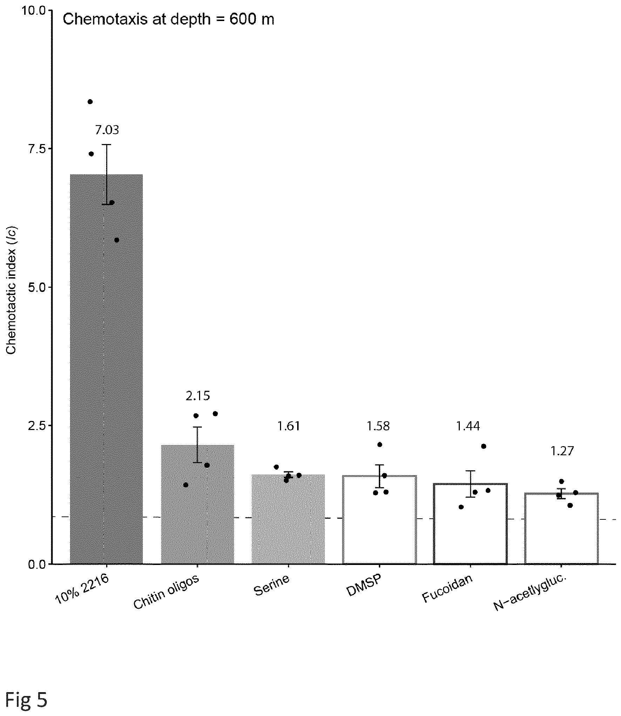

Figure 5 shows a diagram illustrating the in situ chemotactic response of a natural marine microbial population at depth (600 m) after 2h of deployment using the same sampling device toward highly characterized bacterial chemotattractants (10% Marine Broth 2216, 1 mM serine and 1 mM DMSP) and natural constituents of marine nutrient patches and particles (1 mM N-acetylglucosamine, 10 mg/ml fucoidan and 10 mg/ml chitin oligosaccharides). The chemotaxis index /c represents the strength of attraction of the bacteria community towards a compound compared to a negative control of ultrafiltered seawater. It is calculated by normalizing the cell counts obtained in the treatment wells by the mean cell count within the wells containing the filtered seawater negative controls (FLW). Each treatment and control were tested in four ISCA replicates contained in the deployment system. Bacterial cells were quantified by flow cytometry (FSW = 1.34 ± 0.30 × 10⁴; 10% MB2216 = 9.4 ± 1.45 × 10⁴; serine = 2.16 ± 0.13 × 10⁴ ; DMSP = 2.12 ± 0.54 × 10⁴ ; N-acetylglucosamine = 1.7 ± 0.23 × 10⁴; fucoidan = 1.94 ± 0.63 × 10⁴; chitin oligosaccharides = 2.88 ± 0.86 × 10⁴ cells/mL). The concentration of Marine Broth 2216, chitin oligosaccharides and serine deployed at 600 m deep in the sea induced a chemotactic response significantly higher than the filtered seawater (FSW) controls, of *Ic*= 7.03, 2.15 and 1.61 respectively. In all pairwise comparisons: t-test, p < 0.005; Error bars represent SEM

## Claims

1. A sampling device for collecting microorganisms from an environment in particular aqueous environment by chemotaxis comprising:
- a housing,
- at least one electronic control unit interacting with the housing; and
- at least one cast that can be arranged within the housing
wherein the cast is designed to receive at least one assay plate, preferably multiple assay plates;
wherein the at least one assay plate comprises at least one well, preferably up to 20 or more wells, filled with at least one chemoattractant compound and at least one water negative control, and
wherein each well is connected to the outside by at least one port;
wherein the cast is further designed to receive at least one sealing plate at least partially covering the at least one assay plate,
wherein the sealing plate is designed to be moveable between
a first position, wherein the at least one sealing plate seals the port of the at least one well of the assay plate (sealing position / closed position), and
a second position, wherein the at least one sealing plate opens the port of the at least one well of the assay plate (open position),
wherein the cast is connected to the at least one electronic control unit.

2. Sampling device according to claim 1, **characterized in that** the at least one cast is made of pressure and corrosion resistant material, in particular is made of polyoxymethylene (POM).

3. Sampling device according to one of the preceding claims, **characterized in that** the at least one assay plate contains 20 wells or more that each can be filled with at least one chemoattractant compound, preferably 5 wells or more per row and per chemoattractant compound.

4. Sampling device according to one of the preceding claims, **characterized in that** the sealing plate is designed in a grid like manner with alternating slits-forming gaps for open position and bridges for sealed position

5. Sampling device according to one of the preceding claims, **characterized in that** the at least one sealing plate is provided with elements for sealing the ports of each well of the assay plate.

6. Sampling device according to claim 5, **characterized in that** the sealing elements are arranged on the bridges of the sealing plate.

7. Sampling device according to one of claims 5-6, **characterized in that** elements for sealing the ports of each well are evenly spaced for aligning with the evenly spaced port of the wells of the assay plate.

8. Sampling device according to one of claims 5-7, **characterized in that** the at least one sealing element is designed in a spheric shape, in particular in form of a rubber sphere.

9. Sampling device according to one of the preceding claims, **characterized in that** the cast further contains at least one motor, in particular a pressure compensated motor, for moving the at least one sealing plate along the at least one assay plate between the first position and the second position and conversely.

10. Sampling device according to one of the preceding claims, **characterized in that** the at least one electronic control unit is secured on the side of the housing in a titanium enclosure allowing for a fully stand-alone deployment.

11. A method for collecting bacteria from an aquatic environment by chemotaxis employing a sampling device according to one of the preceding claims, wherein the method comprises:
- Providing at least one assay plate with at least one well filled with at least one chemoattractant compound;
- Placing the at least one assay plate in the at least one cast, wherein the at least one assay plate is at least partially covered by the at least one sealing plate such that the ports of the wells of the assay plate are sealed / closed;
- Placing and securing the at least one cast with the at least one sealed assay plate in at least one housing, in particular a modified Niskin bottle;
- Descending the at least one housing with the at least one cast with the at least one sealed assay plate to a predetermined location in a liquid, in particular aqueous environment;
- Closing the housing once the predetermined location is reached,
- Moving the at least one sealing plate automatically from the closed position to the open position to open the ports of the wells of the assay plate;
- Keeping the ports of the wells open for a predetermined time for allowing bacteria to enter the wells of the assay plate through the ports;
- Moving the at least one sealing plate automatically from the open position to the closed position to close the ports of the wells of the assay plate after the predetermined sampling time; and
- Ascending the sampling device.

12. Method according to claim 11, **characterized in that** during descent the at least one housing is open, while the ports of the wells of the assay plates are closed by the sealing plate.

13. Method according to one of claims 11-12, **characterized in that** the at least one housing is closed once the predetermined depth is reached.

14. Method according one of the claims 11-13, **characterized in that** the sampling time is at least 1 hour, preferably at least 2 hours.

15. Method according to the claims 11-14, **characterized in that** the sampling device is deployed in any type of environment containing at least an aqueous phase.
